(19) Europäisches Patentamt
European Patent Office
Office européen des brevets

(11) Publication number: 0 230 119
A1

(12) EUROPEAN PATENT APPLICATION

(21) Application number: 86309440.5

(22) Date of filing: 04.12.86

(51) Int. Cl.4: C 12 N 15/00, C 07 H 21/04, C 12 N 1/20, C 07 K 15/26, C 12 P 21/00

(30) Priority: 04.12.85 US 804815

(43) Date of publication of application: 29.07.87 Bulletin 87/31

(84) Designated Contracting States: AT BE CH DE ES FR GB GR IT LI LU NL SE

(71) Applicant: IMMUNEX CORPORATION, 51 University Building Suite 600, Seattle Washington 98101 (US)

(72) Inventor: Baker, Paul E., 16710 Agate Pass Road, Bainbridge Island Washington 98110 (US)
Inventor: Cerretti, Douglas Pat, 1607 North 197th Place, Seattle Washington 98133 (US)
Inventor: Cosman, David J., 116 11th Avenue East, No. 501, Seattle Washington 98102 (US)
Inventor: McKereghan, Kate N., 8830 233rd Place Southwest, Edmonds Washington 98020 (US)

(74) Representative: Sheard, Andrew Gregory et al, Kilburn & Strode 30, John Street, London WC1N 2DD (GB)

(54) **Cloning, characterization and expression of bovine gamma interferon.**

(57) A chemically-synthesized oligonucleotide composing a portion of the nucleotide sequence of human IFN-γ is employed as a probe to isolate the gene coding for human IFN-γ from a cDNA library prepared form RNA derived from peripheral blood leukocytes. Double stranded cDNA is prepared from the polyadenylated RNA extracted from bovine cells thought to produce IFN-γ. Such cDNA is inserted within a plasmid vector and the recombinant plasmid employed to transform hosts. Plasmid DNA, prepared from the pools of the transformed hosts, is hybridized with the human IFN-γ gene probe. Pools of host cells that provide a positive signal to the human cDNA probe are identified, subdivided and rescreened until a single positive colony is identified. Bovine plasmid cDNA is prepared from this colony, and the bIFN-γ gene is sequenced. The coding region for the bIFN-γ gene is inserted into an expression vector for production of functional bIFN-γ.

EP 0 230 119 A1

IMMUNEX CORPORATION

Application 0602

# CLONING, CHARACTERIZATION AND EXPRESSION OF BOVINE GAMMA INTERFERON

## Technical Field

The present invention relates to bovine gamma interferon (hereinafter "bIFN-γ") and, more particularly, to the cloning of the gene coding for bIFN-γ by use of a probe derived from a human IFN-γ complementary deoxyribonucleic acid ("cDNA") clone to screen a cDNA library synthesized from bovine messenger ribonucleic acid ("mRNA") containing bIFN-γ mRNA.

## Background of the Invention

A serious problem facing the cattle industry is that during shipment, for instance from grazing areas to feed lots, a significant proportion of the cattle lose weight and become sick or even die. It is believed that the stress that cattle undergo during shipment causes elevation in steroid hormone production which leads to a diminution of immune responsiveness. When animals arrive at a feed lot, due to decreased immune reactivity, they fall prey to common bacterial and viral infections which, under normal situations, the animals have the ability to counteract. Various types of upper respiratory tract infections may occur which may result in serious illness or even death from these normally rejected disease-causing infectious agents.

Currently, considerable attention has been focused on the possibility of combating this "shipping fever" syndrome with various species of interferon. Studies have shown that two species of interferon, (IFN-α) and (IFN-β), exhibit potent antiviral activity. These interferons may prove useful against certain viral infections to cattle. A third interferon species, IFN-γ, has been shown to be not only a potent viral inhibitor, but also capable of regulating activity on cells of the immune system at much lower concentrations than with IFN-α and IFN-β. For instance, IFN-γ up-regulates functions known to beneficially influence phagocytosis of opsonized microorganisms, such as expression of Fc receptors, Guyre et al., J. Clin. Invest., 72:293 (1983). In addition, Nathan et al. in J. Exp. Med., 159:670 (1983) reported that IFN-γ can

inhibit growth of intracellular parasites by augmenting elaboration of oxygen intermediates. Further, Celada et al. in J. Exp. Med, 160:55 (1984) reported inducement of macrophage-mediated tumor toxicity with low doses of IFN-γ.

Accordingly, the availability of large quantities of bIFN-γ may provide new methods of combating diseases of cattle. Heretofore, bIFN-γ has been produced by stimulating bovine lymphocyte cells with a mitogen. Unfortunately, this "natural" source of bIFN-γ has not proven to be capable of generating sufficient quantities of homogeneous IFN-γ to thoroughly investigate its potential therapeutic usefulness.

One potential method of producing relatively large quantities of homogeneous bIFN-γ is through recombinant DNA techniques. Recombinant DNA techniques have been developed for economically producing a desired protein once the gene coding for the protein has been isolated and identified. A discussion of such recombinant DNA techniques for protein production is set forth in the editorial and supporting papers in Volume 196 of Science (April, 1977). However, to take advantage of the recombinant DNA techniques discussed in this reference, the gene coding for bIFN-γ must first be isolated.

## Summary of the Invention

In accordance with the present invention, the gene coding for bIFN-γ is isolated from a cDNA library with a nick-translated human cDNA probe. The probe is isolated from a human cDNA library by use of a synthetic oligonucleotide probe corresponding to a portion of the nucleotide sequence of human IFN-γ. Total bovine RNA is extracted from lymph node cells known to produce relatively high levels of bIFN-γ. Polyadenylated mRNA is isolated from the total RNA extract. A cDNA library is constructed by reversed transcription of the polyadenylated mRNA with reverse transcriptase. The DNA is rendered double-stranded with DNA polymerase I and inserted into an appropriate cloning vector. Resultant recombinant cloning vectors are used to transform an appropriate host.

Transformed hosts are identified and grouped into pools. Plasmid DNA prepared from these pools is hybridized with the human cDNA probe that has been radiolabeled. The pool(s) of clones that give a positive signal to the probe are identified and then the putative pool subdivided and the hybridization screen repeated. A single transformant corresponding to the bIFN-γ gene is eventually identified. Plasmid DNA is prepared from this transformant and characterized by DNA sequencing. In addition, the corresponding amino acid sequence is determined from the nucleotide sequence. The coding region of the bIFN-γ gene is cloned into a bacterial expression system to express mature

bIFN-γ. Thereafter, biological assays are conducted to confirm that the expressed protein product is bIFN-γ.

Brief Description of the Drawings

The details of typical embodiments of the present invention will be described in connection with the accompanying drawings, in which:

FIGURE 1 illustrates a major portion of the nucleotide sequence of human IFN-γ isolated with a synthetic oligonucleotide probe. The numbers refer to nucleotide position. The portion of the cDNA fragment used to probe bovine library of cDNA is shown in the box.

FIGURE 2 illustrates the amino acid sequence (lower line) and nucleotide sequence (upper line) of the bIFN-γ gene, with the mature protein beginning at the asterisk. The numbers above each line refer to amino acid positions beginning at the Met residue and the numbers below each line refer to nucleotide position.

FIGURE 3 illustrates the ΔpLBγIFN plasmid, with the coding region of the bIFN-γ gene inserted therein, for use in transforming bacterial host cells to express functional bIFN-γ.

Description of the Invention

Sources of bIFN-γ Producing Cells

Preferably, a cDNA library, from which the gene coding for bIFN-γ will be sought, is constructed from cells previously found to produce relatively high levels of bIFN-γ. These sources may include sources of bovine T-cell tissues, e.g., lymph node or spleen.

Activated bovine peripheral blood also potentially may be a source of bIFN-γ molecules. For use in the present invention, the mononuclear cells can be separated from whole blood by standard techniques, such as by Ficoll-Hypaque centrifugation. Harvested leukocytes are multiplied by standard culturing techniques culturing in vitro in a serum containing medium together with an activating agent, such as a T-cell mitogen.

Preparation of RNA from bIFN-γ Producing Cells

Total RNA from bovine cells, potentially producing IFN-γ is extracted by standard methods, such as disclosed by Chirgwin et al., Biochemistry, 18:5294 (1979), and Maniatis et al., Molecular Cloning, a Laboratory Manual, Cold Spring Harbor Laboratory, Cold Spring Harbor, New York (1982).

As is well known, when extracting RNA from cells, it is important to minimize ribonuclease ("RNase") activity during the initial stages of extraction. One manner in which this is accomplished is to denature the cellular protein, including the RNase, at a rate that exceeds the rate of RNA hydrolysis by RNase. In the procedures of Chirgwin et al., supra, and Maniatis et al., supra at 196, this is carried out by use of guanidinium thiocyanate, together with a reducing agent, such as 2-mercaptoethanol (to break up the protein disulfide bonds). The RNA is isolated from the protein by standard techniques, such as phenol/chloroform extraction, ethanol precipitation or sedimentation through cesium chloride. Alternatively, the RNA can be separated from the protein by extraction with guanidine hydrochloride followed by extraction with phenol/chloroform.

Next, polyadenylated mRNA is separated from the extracted protein. Although several techniques have been developed to carry out this separation process, one preferred method is to chromatograph the polyadenylated mRNA on oligo (dT)-cellulose as described by Edmonds et al., Proc. Natl. Acad. Sci., 68:1336 (1971); Aviv and Leder, Proc. Natl. Acad. Sci., 69:1408 (1972); and Maniatis et al., supra at 197. The oligo (dT)-cellulose column is prepared with a loading buffer and then the mRNA applied to the column. Thereafter, the column is initially washed with a buffer solution to remove the unpolyadenylated mRNA and then the polyadenylated mRNA is eluted from the column with a buffered, low ionic strength eluent. The integrity of the polyadenylated mRNA is verified by gel electrophoresis.

Preparation of cDNA from mRNA

A library of double-stranded cDNA corresponding to the mRNA, as prepared and assayed above, is constructed by known techniques employing the enzyme reverse transcriptase. One such procedure which may be employed in conjunction with the present invention is detailed by Maniatis et al., supra at 230, as modified by Gubler and Hoffman Gene, 25:263-269 (1983). Briefly, the polyadenylated mRNA is reverse transcribed by using oligo-dT, that has been hybridized to the polyadenylated tail of the mRNA, as a primer for a first cDNA strand. The second cDNA strand is synthesized using the enzymes DNA polymerase I, RNase H and E. coli DNA ligase. This procedure eliminates the S1 nuclease mediated cleaving of the hairpin loop formed in the 3' end of the initial cDNA strand, as would be required if standard cDNA synthesis techniques disclosed in Maniatis et al. supra were simply used. The double-stranded cDNA is fractionated by any convenient means to remove the shorter strands, thereby avoiding the needless cloning of small cDNA fractions.

It is to be understood that in accordance with the present invention, alternative standard procedures may be employed to prepare double-stranded cDNA from mRNA. One such alternative technique is disclosed by

Land et al., Nucl. Acids Res., 9:2251 (1981). In the Land et al. protocol, the hairpin loop also is not used as a primer for the second cDNA strand. Rather, the 3' end of the first cDNA strand is tailed with dCMP residues using terminal deoxynucleotidyl transferase ("TdT"). This produces a 3' tail of poly-C residues. Then the synthesis of the second strand is primed by oligo-dG hybridized to the 3' tail. This technique is said to help avoid losing portions of the 5' tail of the second cDNA strand which might occur if the hairpin is cleaved with S1 nuclease, as in the Maniatis et al. protocol.

Cloning of cDNA

Next, the double-stranded cDNA is inserted within a cloning vector which is used to transform compatible prokaryotic or eukaryotic host cells for replication of the vector. Thereafter, the transformants are identified and plasmid DNA prepared therefrom.

To carry out the present invention, various cloning vectors may be utilized. Although the preference is for a plasmid, the vector may be a bacteriophage or a cosmid. If cloning occurs in mammalian cells, viruses also can be used as vectors.

If a plasmid is employed, it may be obtained from a natural source or artificially synthesized. The particular plasmid chosen should be compatible with the contemplated transformation host, whether a bacteria such as E. coli, yeast, or other unicellular microorganism. The plasmid should have the proper origin of replication for the particular host cell to be employed. Also, the plasmid should have a phenotypic property that will enable the transformed host cells to be readily identified and separated from cells that do not undergo transformation. Such phenotypic characteristics can include genes providing resistance to growth inhibiting substances, such as an antibiotic. Plasmids are commercially available that encode genes resistant to various antibiotics, including tetracycline, streptomycin, sulfa drugs, penicillin and ampicillin.

If E. coli is employed as the host cell, many possible cloning plasmids are commercially available which may be used in conjunction with the present invention. A preferred plasmid for performing the present invention is pBR322. This plasmid has been fully sequenced, as set forth in Sutcliffe, Cold Spring Harbor Symp. Quant. Biol., 43:77 (1979). A significant advantage of this plasmid is that it has 11 known unique restriction sites, including the Pst I site in the ampicillin-resistant gene. This feature is particularly useful for cloning by the homopolymer tailing method.

If a bacteriophage is used instead of a plasmid, such phages should have substantially the same characteristics noted above for selection of plasmids. This includes the existence of a phenotypic marker and ligatable termini for attachment of foreign genes.

Preferably, in the present invention, the double-stranded cDNA, having blunt ends, may be inserted into a plasmid vector by homopolymeric tailing. As is well known in the art, in this technique, complementary homopolymer tracks are added to the strands of the cDNA and to the plasmid DNA. The vector and double-stranded cDNA are then joined together by hydrogen bonding between complementary homopolymeric tails to form open, circular hybrid molecules capable of transforming host cells, such as E. coli.

In one procedure for homopolymeric tailing, approximately 50 to 150 dA nucleotide residues are added to the 3' ends of linearized plasmid DNA. A similar number of dT nucleotide residues are added to the 3' ends of the double-stranded cDNA and then the cDNA and plasmid joined together.

In an alternative and preferred method, dG tails are added to the 3' ends of the cloning vector that has been cleaved with an appropriate restriction enzyme. For instance, if the pBR322 plasmid is employed, the restriction enzyme Pst I may be used to digest the plasmid at the ampicillin resistant gene. Complementary dC tails are added to the 3' ends of the double-stranded cDNA prior to insertion of the cDNA segment in the plasmid with an appropriate annealing buffer.

It is to be understood that the double-stranded cDNA may be inserted within plasmid cloning vectors by other various standard methods. One such alternative technique involves attaching synthesized nucleotide linkers to the ends of the cDNA strands by using DNA ligase. The linkers are cleaved with a restriction enzyme to generate cohesive termini for insertion within a plasmid cleaved with the same restriction enzyme. Scheller et al., Science, 196:177-180 (1977); Maniatis et al., supra at 219.

The recombinant DNA plasmids, as prepared above, are used to transform host cells. Although the host may vary, it is preferable that the procaryote E. coli be used for cDNA cloning. Whatever host is chosen, it should not contain a restriction enzyme that would cleave the recombinant plasmid.

If E. coli is employed as a host, preferable strains are MM294 and RR1 (American Type Culture Collection, 12301 Park Lawn Drive, Rockville, MD 20852, USA ["ATCC"] No. 31343). Protocols for transformation of the MM294 host by a plasmid vector are well known, as set forth in Maniatis et al., supra at 255; and, Hanahan, J. Mol. Biol., 166:557 (1983). Protocols for transformation of the RR1 host by a plasmid vector are also well known as set forth in Bolivar et al., Gene, 2:95 (1977) and Peacock et al., Biochem. Biophys. Acta., 655:243 (1981). Other restriction endonuclease negative (R-) strains of E. coli which also could serve as suitable hosts include DH1 ATCC No. 33849 and C600. These strains and the MM294 and RR1 strains are widely commercially available.

In transformation protocols, including those disclosed by Maniatis et al., *supra*, and Hanahan, *supra*, only a small portion of the host cells are actually transformed, due to limited plasmid uptake by the cells. The cells that have been transformed can be identified by placing the cell culture on agar plates containing suitable growth medium and a phenotypic identifier, such as an antibiotic. Only those cells that have the proper resistance gene (e.g., to the antibiotic) will survive. If the recombinant pBR322 plasmid is used to transform E. coli strain MM294 or RR1, transformed cells can be identified by using tetracycline as the phenotypic identifier.

Preparation of Radiolabeled cDNA Screening Probe

A radiolabeled DNA fragment composed of several hundred base-pairs ("bp") corresponding to a majority of the nucleotide sequence of the gene coding for the human IFN-γ species is used as a probe to screen the above-prepared bovine cDNA library. The probe is isolated from a human cDNA library with a radiolabeled, synthetic oligonucleotide probe corresponding to a portion of the nucleotide sequence of human IFN-γ.

To isolate the cDNA probe for use in the screening procedure of the present invention, a human cDNA library is initially prepared from human mRNA. The mRNA is prepared from mononuclear cells that have been separated from whole blood by standard techniques, such as by Ficoll-Hypaque centrifugation. Harvested leukocytes are multiplied by culturing *in vitro* in a serum containing medium together with an activating agent. After a suitable period of time, the cells are harvested by centrifugation. Examples of activating agents that may be used include: the T-cell mitogens phytohemagglutinin ("PHA"), pokeweed mitogen ("PKM") or concanavalin A ("Con A"); E. coli LPS; and, staphylococcal enterotoxin B ("SEB"). Total mRNA from the human leukocyte cells is extracted by standard methods as discussed above, for instance, by the use of guanidinium thiocyanate together with 2-mercaptoethanol. Thereafter, polyadenylated mRNA is separated from the extracted protein by chromatography on oligo (dT)-cellulose.

A library of double-stranded cDNA corresponding to the human mRNA is constructed, as discussed above, by employing reverse transcriptase to form an initial cDNA strand by using the mRNA as a template. Next, the enzyme DNA polymerase I is used to synthesize the second cDNA strand, employing the first strand as a template. The double-stranded cDNA is inserted within a cloning vector which is used to transform compatible host cells for replication of the vector. Preferably, the vector is composed of a plasmid having a number of unique restriction sites, such as the plasmid pBR322. The cDNA prepared from the mRNA may be inserted within this plasmid by homopolymeric tailing, as described above. The recombinant plasmids are used to transform a compatible host, such as a strain of E. coli. Of course, other appropriate hosts may be employed. The host cells that are transformed by the recombinant plasmid are identified with an appropriate standard phenotypic identifier, such as an antibiotic.

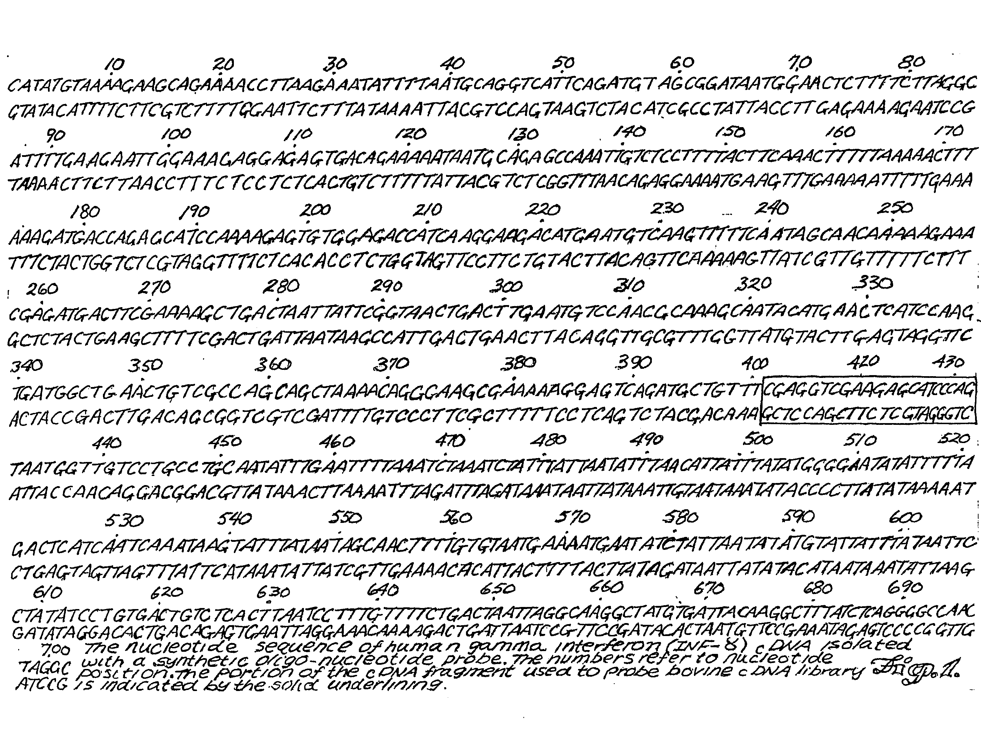

A radiolabeled oligonucleotide is synthesized for use as a probe to screen the human cDNA library. The probe, derived of a portion of the antisense strand of the gene coding for human IFN-γ, has the following composition: 5'CTGGGATGCTCTTCGACCTCG. This probe complements the nucleotides Nos. 569 through 598 of the sense strand shown in FIGURE 1, and has the advantage of being short enough to be relatively easily synthesized, while being long enough to contain sufficient information to be useful as a probe for the human IFN-γ gene. It is to be understood, however, that the composition of the probe may correspond to other portions of the human IFN-γ gene without departing from the scope or spirit of the present invention.

The synthetic oligonucleotide probe may be readily chemically synthesized by well-known techniques, such as by phosphodiester or triester methods. The details of the triester synthesis technique are set forth, for example, in Sood et al., Nucl. Acid Res., 4:2557 (1977); and, Hirose et al., Tet. Lett., 28:2449 (1978). After synthesis, the oligonucleotide probe is labeled with T4 polynucleotide kinase and $^{32}P$-ATP. A standard protocol for the labeling procedure is set forth in Maniatis et al., supra at 122. Advantageously, the oligonucleotide probe can be synthesized with OH 5' termini, thereby avoiding the phosphatase procedure typically required.

The human cDNA library is screened with the synthetic radiolabeled probe as detailed infra, for example, in Examples 3 and 4. Plasmid DNA is then prepared from the particular positive colony identified by the screening procedure. By restriction endonuclease digestion the isolated plasmid DNA was found to include a major portion of the coding region of the human IFN-γ gene.

The isolated human plasmid DNA was used as a probe for screening the human cDNA library prepared above. A probe of this relatively large size increases the likelihood that cDNA, actually coding for bIFN-γ would be hybridized rather than non-IFN-γ coding cDNA fragments. As detailed below, use of this probe was successful in isolating the bIFN-γ gene from a cDNA library. It is to be understood that probes corresponding to other portions of the nucleotide sequence of the human plasmid DNA fragment may be employed without departing from the spirit or scope of the present invention.

The human cDNA probe is radiolabeled prior to being used for hybridizing to the bovine cDNA library pools. Due to the relative large size of the probe, various labeling techniques may be employed; however, preferably the probe is labeled by "nick translation." In this well-known technique, as discussed by Rigby et al., J. Molec. Bio., 113:237 (1977), and Maniatis et al., supra at 108, nicks are introduced at widely separated sites in the DNA by very limited treatment with DNase I, thereby exposing a free 3'-OH group at each nick. DNA polymerase I is employed to incorporate appropriate radiolabeled deoxynucleotide triphosphates ($^{32}$P-dNTPs), at the 3'-OH terminus and concurrently remove the nucleotide from the 5' side of the nick causing sequential movement of the nick along the DNA ("nick translation").

Screening of cDNA Library

In the screening procedure of the present invention, the transformants are initially pooled into relatively large groups each composed of approximately 2,500 transformants. The replicated plasmids are extracted from the transformants using any one of several well-known techniques, such as by alkaline lysis. Plasmid DNA is prepared by cleaving the extracted plasmids with appropriate restriction enzymes. The resulting DNA segments are fractionated by electrophoresis on agarose gels and then directly analyzed by Southern blotting as described by Southern, J. Mol. Biol., 98:503 (1975). The DNA fragments that bind to the nitrocellulose filter in the Southern blotting procedure are hybridized with the labeled cDNA probe. The specific DNA fragments that hybridize to the probe are identified by autoradiography.

The putative pool(s) of clones that discloses a strongly hybridizing band during autoradiography is subdivided into groups of approximately 500 transformants, and then the above-described hybridizing screen using the labeled human cDNA probe is repeated. This process of subdividing putative pools of clones and screening transformants is repeated until a desired pool size is obtained. A single transformant that hybridizes to the labeled probe is then identified by the well-known colony hybridizing technique of Grunstein and Hogness, Proc. Natl. Acad. Sci. (USA), 72:3961 (1975). By this procedure, applicants have discovered one such positive colony. Plasmid DNA, designated as pBYIFN-7, is prepared from this particular colony.

Characterization of Screened cDNA

The plasmid DNA prepared above is sequenced using standard chain-termination methods. This technique of nucleotide sequencing was originated by Sanger et al., Proc. Natl. Acad. Sci. (USA), 70:5463 (1977). See U.S. Patent No. 4,322,499. Methods for chain-termination sequence determination are set forth in the Amersham Handbook entitled, M13 Cloning and Sequencing, Blenheim Cresent, London (1983) (hereinafter "Amersham Handbook"); Messing, 2 Recombinant DNA Technical Bulletin, NIH Publication No. 79-99, 2, 43-48 (1979); Norrander et al., Gene, 26:101 (1983); Cerretti et al., Nucl. Acids Res., 11:2599 (1983); and, Biggin et al., Proc. Natl. Acad. Sci. (USA), 80:3963 (1983). M13 filamentous phage is employed as a vector to clone the DNA sequence of interest. These phage vectors provide single-stranded DNA templates which are readily sequenced by the chain-termination method, which involves priming a single-stranded template molecule with a short primer strand having a free 3' hydroxyl group and then using DNA polymerase (Klenow fragment) to copy the template strand in a chain extension reaction using all four deoxyribonucleotide triphosphates, i.e., dATP, dCTP, dGTP, and dTTP (collectively referred to as "dNTPs"), with one of the dNTPs being radiolabeled. In the synthesis reaction, a nucleotide specific chain terminator lacking a 3'-hydroxyl terminus, for instance, a 2', 3' dideoxynucleotide triphosphate ("ddNTP"), is used to produce a series of different length chain extensions. The terminator has a normal 5' terminus so that it can be incorporated into a growing DNA chain, but lacks a 3' hydroxyl terminus. Once the terminator has been integrated into a DNA chain, no further deoxynucleotide triphosphates can be added so that growth of the chain stops. Four separate synthesizing reactions are carried out, each having a ddNTP of one of the four nucleotide dNPTs, i.e., dATP, dCPT, dGTP and dTTP. One of the normal dNTPs is radiolabeled so that the synthesized strands, after having been sorted by size on a polyacrylamide gel, can be autoradiographed. The chain extensions from the four reactions are placed side by side in separate gel lanes so that the pattern of the fragments from the autoradiography corresponds to the nucleic acid sequence of the cloned DNA.

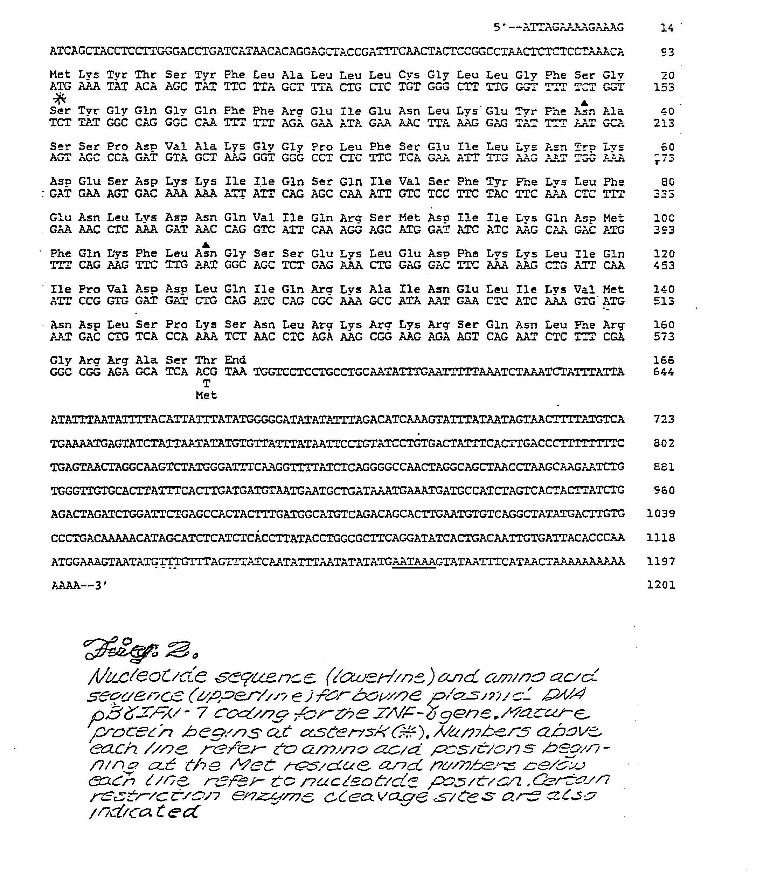

FIGURE 2 illustrates the nucleotide sequence of the bIFN-γ gene contained in the pBγIFN-7 plasmid DNA prepared above with the nucleotides numbered from the beginning of the 5' terminal. The corresponding amino acid composition of the gene is also illustrated in FIGURE 2, with the residues numbered from the beginning of the coding region of the gene (nucleotide No. 94). Applicants believe that the mature protein begins at the Ser residue, No. 21 (nucleotide No. 154), indicated with an asterisk, and extends to the Thr residue, No. 165 (nucleotide No. 589).

In preparation for the sequencing procedures, the plasmid DNA containing the DNA insert is subcloned into M13 phage vectors to form single stranded DNA templates. A universal primer is used to sequence the sense and antisense strands. Rather than relying on the sequencing results obtained from sequencing the entire length of the fragments with a single chain-termination procedure, an additional synthetically produced primer is used to initiate the chain-termination procedure from an intermediate location along the length of the subcloned DNA fragment. The composition of the synthetically produced primer was based on the sequence information obtained using the universal primer. By this process, both strands of the subcloned DNA fragment are sequenced in overlapping fashion, thereby serving to redundantly confirm the sequences.

It is to be understood that rather than employing the chain-termination technique outlined above, other known methods may be utilized to sequence cloned bovine cDNA inserts without departing from the spirit or scope of the present invention. For instance, the chemical degradation method of Maxam and Gilbert as set forth in Proc. Nat'l Acad. Sci. (USA), 74:560 (1977) can be used.

Expression of Functional bIFN-γ from cDNA Clones

To determine whether the cDNA coding region of the bIFN-γ gene as contained in plasmid pBγIFN-7 would encode functional bIFN-γ, the gene is expressed in an expression system and then tested in a virus plaque reduction assay. cDNA corresponding to the coding region of the IFN-γ gene is inserted into an expression vector designed to direct synthesis of the mature form of bIFN-γ from host cells. Preferably, but not essential, a plasmid expression vector is employed for expression in bacteria host cells. Ideally, the plasmid vector contains the λ phage $P_L$ promoter and the bacteria host, for instance a strain of E. coli contains a thermolabile cI repressor of $P_L$ transcription. In addition, if E. coli is employed as the host, preferably the expression vector contains an origin of replication, for example from plasmid pBR332, for high copy DNA replication and an ampicillin resistant gene ("$Amp^r$"), also from plasmid pBR332 for convenient selection of transformed E. coli hosts. Examples of expression vectors meeting these requirements include: plasmid pPL (Pharmacia Fine Chemicals, Cat. No. 27-4946-01); and, plasmid pPLc28 (ATTC No. 53082).

If E. coli is used as a host, to enhance the level of expressed bIFN-Υ, it is desirable that a highly efficient ribosome binding site is employed upstream from the bIFN-Υ cDNA. The sequence for the ribosome binding site can be based, for instance, on the sequence that has been used for expression of human IFN-Υ, Tessier et al., Nucl. Acids Res., 12:7663 (1984). The ribosome binding site sequence is incorporated in a synthetic oligonucleotide set forth in TABLE 1 below. The synthetic oligonucleotide may be synthesized by triester methods as detailed by Sood et al., supra and Hirose et al., supra, or by phosphodiester methods.

TABLE 1

```
                                Met  Ser  Tyr  Gly
         ***
5' - CGATAACACAGGAACAGATCT      ATG  TCT  TAT  GG   - 3'
  3'    TATTGTGTCCTTGTCTAGA     TAC  AGA  ATA  CC   - 5'
     Cla I             Bgl II
```

As illustrated in TABLE 1, the synthetic oligonucleotide is constructed with a cohesive Cla I 5' end and a blunt 3' end which contains the initiation codon ATG and sequences encoding the first three amino acid residues (Ser, Tyr, Gly) of the mature bIFN-Υ protein. The portion of the oligonucleotide between the Cla I restriction enzyme cleavage site and the Met initiation codon composes the ribosome binding site sequences which include a stop codon (marked by the asterisks), a Shine-Dalgarno region (shown in dotted line) and a Bgl II restriction enzyme cleavage site. It is to be understood that the 5' terminus and the portion of the oligonucleotide between the 5' terminus and the Met initiation codon may be of various other compositions to correspond with the construction of the particular plasmid in which the oligonucleotide, together with the bIFN-Υ gene, is being ligated.

Assay for bIFN-Υ

After transformation of the bacteria hosts with the IFN-Υ gene containing expression vector, and upon heat induction, expression of bIFN-Υ is confirmed with a plaque reduction assay. The details of this type of assay are discussed in Langford et al., Meth. Enzym., 78:339 (1981). Briefly in the assay serial $\log_2$ dilutions of recombinant expression product are cultured in 50 microliters ("ul") of Roswell Park Memorial Institute-1640 Medium ("RPMI-1640") in 10% (v/v) fetal bovine serum in individual wells of 96-well microtiter plate (Corning Cat No. 25860). The last well in each row serves as a medium control and, thus, did not contain any expression product. Also, human

IFN-α of known activity served as a positive control and was diluted in serial $\log_2$ in the same manner as the samples.

Madin-Darby bovine kidney cells (MDBK, ATCC No. CCL 22) are harvested from log phase growth by trypsinization and washed free of growth medium. $3 \times 10^4$ MDBK cells in 100 ul of RPMI-1640 in 10% (v/v) fetal calf serum ("FCS") is placed in each well. The microplates are incubated for 24 hours at 37°C in a humidified atmosphere of 5% $CO_2$ in air. The medium is then removed from the wells. A thawed aliquot of vesicular stomatitis virus ("VSV"), previously quantified for infectious particles, is diluted and 30-35 infectious particles in 100 ul of the same medium as above is placed in each well. After one hour of incubation with gentle rocking at 37°C, the unattached virus is removed from each well. Thereafter, each well was overlayed with 100 ul of 0.5% methyl cellulose in the same medium as above. After an additional 18-24 hours of incubation at 37°C, the methyl cellulose is removed and the wells are stained for three minutes with 0.5% crystal violet in 70% methanol. The stain is then removed by immersing the microtiter plate in water several times.

Thereafter, the plaques are counted. Interferon activity of individual samples is calculated according to the following formula:

$$PDD_{50} = DL + \frac{[(P_{50} - P_L)(DH-DL)]}{P_H - P_L}$$

where:

| | |
|---|---|
| $PDD_{50}$ = | the 50% plaque-depressing dose, |
| DL = | reciprocal of the lower bracket of the 50% dilution, |
| DH = | reciprocal of the higher bracket of the 50% dilution, |
| $P_H$ = | the number of plaques at the higher dilution bracketing the 50% end point, |
| $P_L$ = | the number of plaques at the lower dilution bracketing the 50% end point, |
| $P_{50}$ = | the number of plaques at the 50% end point, which is: $\frac{\text{mean no. plaques in all medium only wells}}{2}$ |

By use of the present assay, the expression system is found to generate high levels of bIFN-γ activity, i.e., 115,300 units per milliliter ("U/ml"). Only background activity (2 U/ml) is detected in controlled plasmids lacking the bIFN-γ sequences. This confirms that the gene isolated by applicants, as set forth in FIGURE 2, coincides with the bIFN-γ gene.

The processes and products of the present invention are further illustrated by the following examples.

## EXAMPLE 1

### Preparation of Polyadenylated mRNA

Bovine retropharyngeal lymph node cells (from Weber's Meat Packing, Sumner, Washington), at a concentration of approximately $10^7$ cells per milliliter, were cultured in RPMI-1640 medium together with 10% (v/v) FCS and the mitogenic lectin Con A at a concentration of 7.5 micrograms per milliliter ("ug/ml"). The cells were cultured for approximately 17 hours in a humidified atmosphere of 5% $CO_2$ in air. After this period of time, viable cells were harvested by centrifugation.

Total RNA was extracted from the mononuclear cells essentially by the method as described by Chirgwin et al., supra. In the procedure guanidinium thiocyanate was used to denature the cellular protein including the RNase at a rate that exceeds the rate of RNA hydrolysis by RNase. The mRNA was removed from the cellular protein by ethanol precipitation followed by resuspension (extraction) with 8 M guanidine HCl, 25 mM sodium acetate. Guanidine HCl extracted RNA was then reextracted with an equal volume of phenol/chloroform/isoamyl alcohol (25 volumes/24 volumes/1 volume). The aqueous phase containing the RNA resulting from such extraction process was then rendered 50 mM sodium acetate, and precipitated by addition of 0.6 volume ethanol. RNA was collected by freezing at -20°C followed by centrifugation.

Thereafter, polyadenylated mRNA was separated from the extracted protein on an oligo (dT)-cellulose chromatography column using the method disclosed by Maniatis et al., supra at 197. Briefly, the column was prepared with application buffer composed of 20 mM Tris-HCl (pH 7.6), 0.5 M NaCl, 1 mM ethylene diamine tetraacetate ("EDTA") and 0.1% sodium dodecyl sulfate ("SDS"). The RNA pellet was dissolved in water and application buffer and then loaded onto the column. The nonadsorbed material was eluted by initial washings with application buffer followed by additional washings with application buffer containing 0.1 M NaCl. The retained polyadenylated mRNA was eluted with buffers of reduced ionic strength composed of 10 mM Tris-HCl (pH 7.5), 1 mM EDTA and 0.05% SDS. The eluted polyadenylated mRNA was precipitated at -20°C with 1/10 volume sodium acetate (3M, pH 5.2) and 2.2 volumes of ethanol. After elution of the polyadenylated mRNA from the oligo (dT)-cellulose column, the integrity of the polyadenylated mRNA was confirmed by electrophoresis through agarose gels as detailed in Maniatis et al., supra at 199.

## EXAMPLE 2

### Construction of cDNA Library

A library of double-stranded cDNA corresponding to the mRNA was prepared from the purified mRNA in Example 1 by employing the standard procedure detailed by Maniatis et al., supra at 229 as modified by Gubler and Hoffman, supra. Oligo-dT was hybridized to the polyadenylated tail of the mRNA to serve as the primer for the reverse transcription of the first cDNA strand. The enzyme avian myeloblastosis virus ("AMV") reverse transcriptase synthesized the first cDNA strand by using the mRNA as a template. Briefly, the synthesis of the first cDNA strand is carried out in a reaction volume of 20-40 ul containing 50 mM•Tris HCl [pH 8.3], 10 mM $MgCl_2$, 10 mM dithiothreitol, ("DTT"), 4 mM•Na pyrophosphate, 1.25 mM dGTP, 1.25 mM dATP, 1.25 mM TTP, 0.5 mM dCTP, 15-20 uCi of [$\alpha$-$^{32}$P] dCTP [3,000 Ci/mmol], 100 ug/ml of oligo ($dT_{12-18}$), 150 ug/ml mRNA (from Example 1), 3,000 units AMV reverse transcriptase/ml. The reaction was carried out at 43° C for thirty minutes and then stopped by adding EDTA to 20 mM. The reaction products were extracted with phenol and precipitated with ethanol out of 2 M $NH_4$• acetate, as described by Okayama and Berg, Mol. Cell. Biol., 2:161-170 (1982). The second cDNA strand was synthesized in a reaction containing 100 ul of 20 mM Tris•HCl as above [pH 7.5], 5 mM MgCl, 10 mM $(NH_4)_2SO_4$, 100 mM KCl, 0.15 mMβ-NAD, 50 ug/ml BSA, 40 um dNTPs, 8.5 units/ml of *E. coli* RNase H, 230 units/ml DNA polymerase I, 10 units/ml *E. coli* DNA ligase. This mixture was incubated at one hour for 12° C and then for a further hour at 22° C. Thereafter, EDTA was added to 20 mM to stop the reaction. The resulting double-stranded cDNA was extracted with phenol as described above.

The double-stranded cDNA was fractionated into size classes by Sephacryl S-400 (Pharmacia Fine Chemicals) column chromatography and monitored by analysis using alkaline agarose electrophoresis employing end-labeled fragments of pBR322 DNA as molecular-weight markers. DNA strands having a length of less than 500 bp were culled out to avoid needless cloning of these undersized cDNA fractions.

The double-stranded cDNA fractions, as prepared above, were inserted into the Pst I site of the pBR322 plasmid (Pharmacia Fine Chemicals) using the method disclosed by Maniatis et al., supra, beginning at 239. In this procedure the double-stranded cDNA was tailed with poly (dC) at its 3' ends. The plasmid pBR322 was digested with Pst I endonuclease and then tailed with poly (dG) at its 3' ends. The tailed plasmid DNA and the tailed cDNA were annealed with annealing buffer (0.1M NaCl, 10 mM Tris-HCl (pH 7.8) and 10 mM ETDA) to form novel recombinant plasmids. All restriction enzymes described herein are commercially available from New England Biolabs, Beverly, Massachusetts.

The recombinant plasmids were transformed into E. coli strain MM294 by using the procedure of Hanahan, supra in which the E. coli cells were prepared by growth in elevated levels of $Mg^{2+}$. The transformation hosts were plated and then transformants were identified by use of tetracycline as a phenotypic identifier. By use of this technique, applicants obtained approximately 35,000 independent transformants.

## EXAMPLE 3

Preparation of Human IFN-γ cDNA Screening Probe

Leukocyte concentrates of a volume of 350-400 ml, obtained from human whole blood (mixture from Portland, Oregon Red Cross), were mixed and diluted in $Ca^{++}$, $Mg^{++}$ free phosphate buffered saline ("PBS") layered onto Histopaque (Sigma Chemical Company, St. Louis, MO) and then centrifuged at 600 x g for 30 minutes at room temperature. An interface layer, consisting of leukocytes, was removed, washed with PBS and centrifuged at 400 x g for 10 minutes at room temperature. The cells were washed two more times in $Ca^{++}$, $Mg^{++}$ free PBS and centrifuged at 200 x g for 10 minutes after each washing.

The cells were then added to plastic culture flasks in RPMI-1640 medium together with 10% fetal bovine serum (v/v) and 10 ug/ml ConA (Sigma Chemical, St. Louis, MO). Sixteen hours later, stimulated cells were harvested for RNA.

Total RNA was extracted from the mononuclear cells and polyadenlylated mRNA was separated therefrom on an oligo (dT)-cellulose chromatography column, using the protocols set forth in Example 1. The integrity of the resulting polyadenylated mRNA was confirmed by agarose gel electrophoresis. A library of double-stranded cDNA corresponding to the human mRNA was prepared by the method set forth above in Example 2. The resulting double-stranded cDNA fractions of sizes greater than 500 bp were inserted into the Pst I site of the pBR322 plasmid by the homopolymeric tailing method set forth in Example 2. The recombinant plasmids were transformed into E. coli strain MM294 and then the transformants were identified by use of tetracycline as a phenotypic identifier. By this process, applicants identified approximately $1 \times 10^6$ independent transformants.

A synthetic oligonucleotide probe was chemically synthesized by standard triester method, as detailed by Sood et al., supra, and Hirose et al.,

supra, and then radiolabeled with $^{32}P$ for use in screening the human cDNA library. The probe was composed of the following nucleotide sequence: 5'-CTGGGATGCTCTTCGACCTCG-3', which corresponds with nucleotides 569 through 589 in FIGURE 1. To facilitate labeling, the 5' ends of the oligonucleotides are synthesized with OH termini, thereby eliminating the phosphatase treatment which typically must be employed when labeling DNA fragments. The labeling protocol included adding 1 ul of the synthetic oligonucleotides to 16 ul of $^{32}P$-ATP (7000 Ci/mM), 1 ul (10 U) of T4 polynucleotide kinase and 2 ul of 10 x kinase buffer I (0.5 M Tris-Cl (pH 7.6), 0.1 $MgCl_2$, 50 mM dithiothreitol, 1 mM spermidine and 1mM EDTA). The reaction was carried out at 37°C for thirty minutes, and thereafter the synthesized oligonucleotides were extracted with phenol/chloroform. The labeled probes were separated from unlabeled oligonucleotides by chromatography on or filtration through Sephadex G-50 columns (Pharmacia Fine Chemicals).

To facilitate initial screening of the human cDNA library, the transformed bacterial cultures were grouped into pools, each having approximately 5,000 different clones. Plasmid DNA was removed from samples of the host bacteria by standard alkaline lysis method detailed by Ish-Horowicz and Burke, Nucl. Acids Res., 9:2989 (1981). The isolated plasmids were digested to completion with Pvu II and Hind III standard procedures. Next the plasmid digests were fractionated by electrophoresis through 0.8% agarose gel and then blotted onto nitrocellulose filter by the standard method of Southern, supra. The DNA that bound to the nitrocellulose filter was hybridized with the labeled synthetic oligonucleotide probe using the procedure detailed in Example 4, infra. The putative pool(s) of clones from which hybridizing bands of DNA were obtained was screened by direct colony hybridization with the radiolabeled synthetic probe, and a single positive colony was identified.

Plasmid DNA was prepared from the identified positive colony by the procedures set forth above and then mapped by standard restriction endonuclease digestion procedures. The nucleotide sequence of the human γ-IFN cDNA is shown in FIGURE 1. The 702 bp human IFN-γ cDNA clone shown in FIGURE 1 was used as a probe for screening the bovine plasmid DNA prepared in Example 2 above.

The cDNA nucleotide probe was radiolabeled by nick translation by the standard procedure set forth in Maniatis et al., supra at 108 and discussed above. By this procedure, the probe was labeled to a specific activity of approximately 5 x $10^8$ CPM/ug DNA. Prior to use in screening protocols, the labeled probe was denatured by boiling in water at 100°C. for ten minutes followed by chilling on ice.

## EXAMPLE 4

Screening of cDNA Library

To facilitate initial screening of the cDNA library prepared in Example 2 above, the transformed bacteria cultures were grouped into pools each having approximately 2,500 different clones. Plasmid DNA was removed from samples of the host bacteria by standard alkaline lysis method detailed by Ish-Horowicz and Burke, supra. The isolated plasmids were cleaved with Pst I and then fractionated by electrophoresis through 1.0% agarose gel with markers of appropriate size. The agarose gel was blotted onto nitrocellulose filter using the method described by Southern, supra. After the transfer process, the filter was air dried and baked for two hours at approximately 80°C under a vacuum to bind the DNA fragments to the nitrocellulose.

The bound DNA was next hybridized with the labeled cDNA probe. Briefly, the baked nitrocellulose was incubated at 55°C for 2-4 hours in prehybridization buffer composed of 6 x NaCl/Cit (1 x NaCl/Cit = 0.15 M NaCl/0.015 M sodium citrate, pH 7) containing 0.1% sarcosyl, 5 x Denhardt's solution (1 x = 0.02% Ficoll, 0.02% polyvinyl pyrrolidone, 0.02% BSA) 100 ug/ml denatured salmon sperm DNA (Sigma Type III, sodium salt) and 0.5% Nonidet P40 detergent. The filter was then incubated for 16 hours at 55°C with the $^{32}P$-labeled cDNA probe ($10^6$ cpm/ml) (from Example 3) in hybridizing solution as above. After hybridization, the filter was washed extensively with 6 x NaCl/Cit at room temperature and then for 1 hour at 42°C and then for 1.5 hours at 55°C. After air drying, the filter was subjected to autoradiography at - 70°C.

From the autoradiography, applicants found a number of strongly hybridizing bands. One putative pool of clones from which the plasmid DNA that produced a strongly hybridizing band was obtained was subdivided into pools of approximately 500 transformants and the hybridization screening procedure repeated. The putative subpool from which a strongly hybridizing band of DNA was seen was then plated. The resulting colonies were probed with the radiolabeled nucleotide probe by the well-known methods of Grunstein and Hogness, supra, using the hybridizing conditions described above. By this process, a single positive host colony was identified.

## EXAMPLE 5

Characterization of Screened cDNA

Plasmid, designated as pBγIFN-7 was prepared with cDNA from the identified positive colony by the procedures set forth in Example 4. The cDNA inserts prepared from the plasmid DNA removed from the positive host colony was sequenced by standard chain-termination protocol essentially as described in the Amersham Handbook, supra, with the variations set forth below. The cDNA insert was digested with Pst I and/or Rsa I and then subcloned into strains mp18 and mp19 of the M13 single-stranded filamentous phage vector (Amersham, Arlington Heights, IL). The mp18 and mp19 phage vectors, as set forth in Norrander et al., supra, contain the following unique cloning sites: Hind III; Sph I; Pst I; Sal I; Acc I; Hinc II; Xba I; BamHI; Xma I; Sma I; Kpn I; Sst I; and, EcoRI. The composition of the mp18 and mp19 vectors are identical, with the exception that the order of the above-identified restriction sites are reversed in the mp19 vector so that both strands of the cDNA insert may be conveniently sequenced with the two vectors. The mp18 and mp19 vectors, with a corresponding strand of the cDNA inserted therein, were used to transform E. coli JM107 of the strain K12 (Bethesda Research Laboratories, Bethesda, MD) to produce relicate single-stranded DNA templates containing single-stranded inserts of the sense and antisense strands.

The synthetic universal primer: 5'-CCCAGTCACGACGTT-3' (P-L Biochemicals, Milwaukee, WI), was annealed to the single-strand DNA templates and used to prime DNA synthesis as described above at page 10. Thereafter, the extension fragments were size-separated by gel electrophoresis and autoradiographed from which the nucleotide sequences of the fragments were deduced.

Deoxyadenosine 5' ($\alpha$-[$^{35}$S] thio) triphosphate (hereinafter "dATP [$\alpha$-$^{35}$S]") was used as the radioactive label in the dideoxy sequencing reactions. Also, rather than using the gel set forth at page 36 of the Amersham Handbook, a 6% polyacrylamide gel was employed (6% polyacrylmide gel, 0.4 mm thick, containing 7 M urea, 100 mM Tris borate [pH 8.1], and 2 mM EDTA).

As noted above, the nucleotide sequence of the pB$\gamma$IFN-7 cDNA is illustrated in FIGURE 2. The coding region of the bIFN-$\gamma$ gene extends from nucleotide No. 94 (Met residue) to nucleotide No. 591 (Thr residue), and the mature protein is believed to begin at the amino acid residue (Ser) corresponding to nucleotide No. 154. The corresponding amino acids, as determined by the nucleotide sequence, are set forth above the codons.

**EXAMPLE 6**

Expression of bIFN-$\gamma$ in Bacteria Hosts

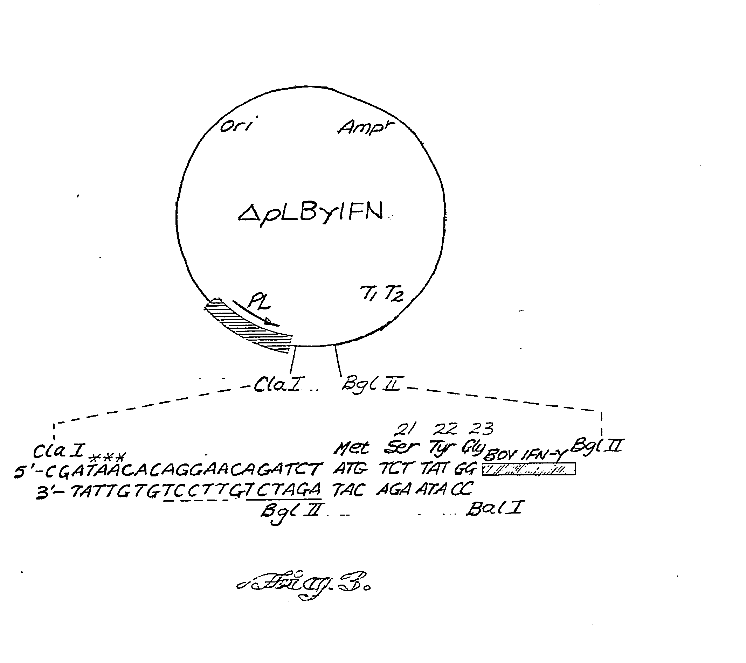

The coding region of the bIFN-$\gamma$ gene shown in FIGURE 2 extending from the Bal I restriction site (nucleotide No. 162) to the Bgl II restriction site (nucleotide No. 966) in the 3' flanking region of the gene was inserted into an expression vector to direct bIFN-$\gamma$ expression in E. coli. This expression vector, designated as ΔpLB λIFN and illustrated in FIGURE 3, was derived from the pBR322 including the origin of replication (ori) and an ampicillin resistance gene ($Amp^r$). The expression plasmid also contains the thermal inducible phage lambda $P_L$ promoter derived from $P_L$-λ (Pharmacia, No. 27-4946-01) (shown in hatched box portion) which functions to direct transcription of bIFN-λ. The expression plasmid also includes the rrnB operon transcriptional terminators, $T_1T_2$, derived from expression vector pKK223-3 (Pharmacia, No. 27-4935-01).

The portion of the coding region of the bIFN-λ gene from the Bal I site (nucleotide No. 162) in FIGURE 2, to the BgL II site (nucleotide No. 966) in the 3' flanking region of the bIFN-λ gene was removed from the pB λIFN-7 cDNA segment illustrated in FIGURE 2 by use of the Bal I and BgL II restriction enzymes in a standard protocol, such as set forth in Maniatis et al., supra at 104. The bIFN-λ gene segment was cleaved from the cDNA clone at the Bal I site, which was located eight nucleotides downstream from the coding region for the mature protein, because no convenient restriction site was found to correspond precisely at the 5' terminal of the coding region for the mature protein.

A synthetic oligonucleotide was chemically synthesized to add back the 5' terminal portion of the coding region of the bIFN-λ gene and also to create a translation initiation codon (ATG). The composition of the oligonucleotide, as shown in Table 1 (supra at page 12) also includes a ribosome binding site upstream from the initiator codon and a Cla I cohesive 5' terminal. The ribosome binding site is based on sequences that give high level expression of human IFN-λ.

Rather than cleaving the coding region of the bIFN-λ gene at the Bal I site, the pB λ IFN-7 cDNA fragment shown in FIGURE 2 could be cleaved at a restriction enzyme site in the 5' flanking region of the gene. Therafter, the nucleotides of the flanking region could be sequentially removed by standard techniques.

The expression plasmid, ΔpLB λIFN (deposited with the ATCC under Accession No. 53,333), was transformed into *E. coli* strain RR1 (ATCC No. 31343) using the standard transformation techniques, such as set forth in Bolivar et al., supra and Peacock et al., supra. This particular strain of *E. coli* contains plasmid pRK248cIts (ATCC No. 33766) which has a gene encoding a thermolabile repressor of the $P_L$ promoter.

Cultures containing the ΔpLB λIFN plasmid in *E. coli* strain RR1 were grown in S.I. medium (M-9 medium) (Maniatis et al., supra) supplemented with 32 g/l tryptone and 20 g/l yeast extract containing M9 salts to 1X, $MgSO_4$

to 0.1 mM, $FeCl_3$ to 0.001 mM and ampicillin at 100 ug/ml overnight at 30°C. They were then diluted 100-fold into S.I. medium without ampicillin and grown to an absorbance at 600 nanometers of 0.7 and then shifted to 42°C for 4 hours to promote derepression. One ml samples of the culture were pelleted by centrifugation at 4°C and then frozen by exposure to a mixture of dry ice and methanol. The pellets were then resuspended in 150 ul of 7 M guanidine hydrochloride and refrozen on dry ice/methanol. The existence of biological activity in the guanidine extract was then ascertained by the plaque reduction assay discussed supra. Applicants found that the ΔpLBγIFN plasmid expressed biological activity of over 1.15 x $10^5$ U/ml. Only background activity of about 2 U/ml was detected in control plasmids which lacked the bIFN-γ gene sequences. This confirms that the pBγIFN-7 cDNA characterized in FIGURE 2 corresponds to the bIFN-γ gene.

As will be apparent to those skilled in the art in which the invention is addressed, the present invention may be embodied in forms other than those specifically disclosed above without departing from the spirit or essential characteristics of the invention. The particular embodiments of the present invention, described above, are therefore to be considered in all respects as illustrative and not restrictive. The scope of the present invention is as set forth in the appended claims rather than being limited to the examples contained in the foregoing description.

In one of its most important aspects, the invention relates to substantially pure DNA that encodes for the expression of bovine interferon gamma. This DNA may substantially completely hybridize to a fragment composed of nucleotide Nos. 1 to 692 of Figure 1.

A polypeptide according to the invention, expressed by such a DNA sequence, may be in mature form or may comprise a cleavable conjugate or signal peptide attached to its N-terminus, and the corresponding DNA may therefore comprise a nucleic acid leader sequence encoding a signal peptide, the leader sequence being disposed 5' to the nucleic acid sequence of the polypeptide.

A DNA sequence encoding bovine interferon gamma may be operably linked with a second DNA sequence capable of effecting its expression.

DNA vectors in accordance with the invention may be designated as cloning vectors or expression vectors.

CLAIMS FOR THE CONTRACTING STATES: BE, FR, DE, GR, IT, LU, NL, CH, LI and GB

1. Substantially pure DNA that encodes bovine gamma interferon.

2. DNA as claimed in Claim 1 and having the nucleic acid sequence of FIGURE 2.

3. DNA as claimed in Claim 1 and having the nucleic acid sequence extending from nucleotide No. 154 to nucleotide No. 591 in FIGURE 2.

4. A recombinant DNA vector comprising a DNA sequence as claimed in Claim 1, 2 or 3.

5. A culture of microorganisms transformed by a recombinant DNA vector as claimed in Claim 4.

6. A polypeptide encoded by the nucleic acid sequence of as defined in any one of Claims 1 to 3.

7. Recombinant bovine gamma interferon expressed by a culture as claimed in Claim 5.

8. A process for preparing recombinant bovine gamma interferon, comprising culturing microorganisms transformed by a recombinant DNA vector as claimed in Claim 4, and recovering bovine gamma interferon.

9. Plasmid pLBγIFN-7, (ATCC 53333).

CLAIMS FOR THE CONTRACTING STATES: AT and ES

1. A process for the preparation of substantially pure DNA that encodes bovine gamma interferon, the process comprising successively coupling appropriate nucleotides in an appropriate sequence.

2. A process as claimed in Claim 1, wherein a complementary strand of nucleic acid serves as a template and the coupling is effected by a transcription enzyme.

3. A process for the preparation of a recombinant vector comprising a DNA sequence that encodes bovine gamma interferon, the process comprising ligating a DNA sequence that encodes bovine gamma interferon into a cleaved vector.

4. A process for the preparation of a culture of microorganisms transformed by a recombinant DNA vector comprising a DNA sequence that encodes bovine gamma interferon, the process comprising transforming at least one microorganism by a recombinant DNA vector comprising a DNA sequence that encodes bovine gamma interferon and culturing the transformed microorganism(s).

5. A process for the preparation of a polypeptide, the process comprising expressing substantially pure DNA that encodes bovine gamma interferon.

6. A process for the preparation of recombinant bovine gamma interferon, the process comprising culturing microorganisms transformed by a recombinant DNA vector comprising a DNA sequence that encodes bovine gamma interferon and recovering bovine gamma interferon.

7. A process as claimed in any one of Claims 1 to 6, wherein the DNA has the nucleic acid sequence extending from nucleotide No. 154 to nucleotide No. 591 in Figure 2.

8. A process as claimed in any one of Claims 1 to 6, wherein the DNA has the nucleic acid sequence extending from nucleotide No. 154 to nucleotide No. 591 in Figure 2.

9. A process for the preparation of plasmid pLBgammaIFN-7 comprising replicating the plasmid identified by ATCC accession No. 53333.

1/3

10 20 30 40 50 60 70 80
CATATGTAAAAGAAGCAGAAAACCTTAAGAAATATTTTAATGCAGGTCATTCAGATGTAGCGGATAATGGAACTCTTTTCTTAGGC
GTATACATTTTCTTCGTCTTTTGGAATTCTTTATAAAATTACGTCCAGTAAGTCTACATCGCCTATTACCTTGAGAAAAGAATCCG

90 100 110 120 130 140 150 160 170
ATTTTGAAGAATTGGAAACGAGGAGAGTGACAGAAAAATAATGCAGAGCCAAATTGTCTCCTTTTACTTCAAACTTTTTAAAAACTTT
TAAAACTTCTTAACCTTTCTCCTCTCACTGTCTTTTTATTACGTCTCGGTTTAACAGAGGAAAATGAAGTTTGAAAAATTTTTGAAA

180 190 200 210 220 230 240 250
AAAGATGACCAGAGCATCCAAAAGAGTGTGGAGACCATCAAGGAAGACATGAATGTCAAGTTTTTCAATAGCAACAAAAAGAAA
TTTCTACTGGTCTCGTAGGTTTTCTCACACCTCTGGTAGTTCCTTCTGTACTTACAGTTCAAAAAGTTATCGTTGTTTTTCTTT

260 270 280 290 300 310 320 330
CGAGATGACTTCGAAAAGCTGACTAATTATTCGGTAACTGACTTGAATGTCCAACGCAAAGCAATACATGAACTCATCCAAG
GCTCTACTGAAGCTTTTCGACTGATTAATAAGCCATTGACTGAACTTACAGGTTGCGTTTCGTTATGTACTTGAGTAGGTTC

340 350 360 370 380 390 400 420 430
TGATGGCTGAACTGTCGCCAGCAGCTAAAACAGGGAAGCGAAAAAGGAGTCAGATGCTGTTTCGAGGTCGAAGAGCATCCCAG
ACTACCGACTTGACAGCGGTCGTCGATTTTGTCCCTTCGCTTTTTCCTCAGTCTACGACAAAGCTCCAGCTTCTCGTAGGGTC

440 450 460 470 480 490 500 510 520
TAATGGTTGTCCTGCCTGCAATATTTGAATTTTAAATCTAAATCTATTTATTAATATTTAACATTATTTATATGGGGAATATATTTTTA
ATTACCAACAGGACGGACGTTATAAACTTAAAATTTAGATTTAGATAAATAATTATAAATTGTAATAAATATACCCCTTATATAAAAAT

530 540 550 560 570 580 590 600
GACTCATCAATCAAATAAGTATTTATAATAGCAACTTTTGTGTAATGAAAATGAATATCTATTAATATATGTATTATTTATAATTC
CTGAGTAGTTAGTTTATTCATAAATATTATCGTTGAAAACACATTACTTTTACTTATAGATAATTATATACATAATAAATATTAAG

610 620 630 640 650 660 670 680 690
CTATATCCTGTGACTGTCTCACTTAATCCTTTGTTTTCTGACTAATTAGGCAAGGCTATGTGATTACAAGGCTTTATCTCAGGGGCCAAC
GATATAGGACACTGACAGAGTGAATTAGGAAACAAAAGACTGATTAATCCGTTCCGATACACTAATGTTCCGAAATAGAGTCCCCGGTTG

700
TAGGC
ATCCG

The nucleotide sequence of human gamma interferon (INF-γ) cDNA isolated with a synthetic oligonucleotide probe. The numbers refer to nucleotide position. The portion of the cDNA fragment used to probe bovine cDNA library is indicated by the solid underlining.

Fig. 1.

5'--ATTAGAAAAGAAAG 14

ATCAGCTACCTCCTTGGGACCTGATCATAACACAGGAGCTACCGATTTCAACTACTCCGGCCTAACTCTCTCCTAAACA 93

```
Met Lys Tyr Thr Ser Tyr Phe Leu Ala Leu Leu Leu Cys Gly Leu Leu Gly Phe Ser Gly    20
ATG AAA TAT ACA AGC TAT TTC TTA GCT TTA CTG CTC TGT GGG CTT TTG GGT TTT TCT GGT   153
 *                                                                       ▲
Ser Tyr Gly Gln Gly Gln Phe Phe Arg Glu Ile Glu Asn Leu Lys Glu Tyr Phe Asn Ala    40
TCT TAT GGC CAG GGC CAA TTT TTT AGA GAA ATA GAA AAC TTA AAG GAG TAT TTT AAT GCA   213

Ser Ser Pro Asp Val Ala Lys Gly Gly Pro Leu Phe Ser Glu Ile Leu Lys Asn Trp Lys    60
AGT AGC CCA GAT GTA GCT AAG GGT GGG CCT CTC TTC TCA GAA ATT TTG AAG AAT TGG AAA   273

Asp Glu Ser Asp Lys Lys Ile Ile Gln Ser Gln Ile Val Ser Phe Tyr Phe Lys Leu Phe    80
GAT GAA AGT GAC AAA AAA ATT ATT CAG AGC CAA ATT GTC TCC TTC TAC TTC AAA CTC TTT   333

Glu Asn Leu Lys Asp Asn Gln Val Ile Gln Arg Ser Met Asp Ile Ile Lys Gln Asp Met   100
GAA AAC CTC AAA GAT AAC CAG GTC ATT CAA AGG AGC ATG GAT ATC ATC AAG CAA GAC ATG   393
                     ▲
Phe Gln Lys Phe Leu Asn Gly Ser Ser Glu Lys Leu Glu Asp Phe Lys Lys Leu Ile Gln   120
TTT CAG AAG TTC TTG AAT GGC AGC TCT GAG AAA CTG GAG GAC TTC AAA AAG CTG ATT CAA   453

Ile Pro Val Asp Asp Leu Gln Ile Gln Arg Lys Ala Ile Asn Glu Leu Ile Lys Val Met   140
ATT CCG GTG GAT GAT CTG CAG ATC CAG CGC AAA GCC ATA AAT GAA CTC ATC AAA GTG ATG   513

Asn Asp Leu Ser Pro Lys Ser Asn Leu Arg Lys Arg Lys Arg Ser Gln Asn Leu Phe Arg   160
AAT GAC CTG TCA CCA AAA TCT AAC CTC AGA AAG CGG AAG AGA AGT CAG AAT CTC TTT CGA   573

Gly Arg Arg Ala Ser Thr End                                                       166
GGC CGG AGA GCA TCA ACG TAA TGGTCCTCCTGCCTGCAATATTTGAATTTTTAAATCTAAATCTATTTATTA   644
                     T
                    Met
```

ATATTTAATATTTTACATTATTTATATGGGGATATATATTTAGACATCAAAGTATTTATAATAGTAACTTTTATGTCA 723

TGAAAATGAGTATCTATTAATATATGTGTTATTTATAATTCCTGTATCCTGTGACTATTTCACTTGACCCTTTTTTTTC 802

TGAGTAACTAGGCAAGTCTATGGGATTTCAAGGTTTTATCTCAGGGGCCAACTAGGCAGCTAACCTAAGCAAGAATCTG 881

TGGGTTGTGCACTTATTTCACTTGATGATGTAATGAATGCTGATAAATGAAATGATGCCATCTAGTCACTACTTATCTG 960

AGACTAGATCTGGATTCTGAGCCACTACTTTGATGGCATGTCAGACAGCACTTGAATGTGTCAGGCTATATGACTTGTG 1039

CCCTGACAAAAACATAGCATCTCATCTCACCTTATACCTGGCGCTTCAGGATATCACTGACAATTGTGATTACACCCAA 1118

ATGGAAAGTAATATGTTTGTTTAGTTTATCAATATTTAATATATATGAATAAAGTATAATTTCATAACTAAAAAAAAAA 1197

AAAA--3' 1201

Fig. 2.

Nucleotide sequence (lowerline) and amino acid sequence (upperline) for bovine plasmid DNA pBγIFN-7 coding for the INF-γ gene. Mature protein begins at asterisk (*). Numbers above each line refer to amino acid positions beginning at the Met residue and numbers below each line refer to nucleotide position. Certain restriction enzyme cleavage sites are also indicated

0230119

3/3

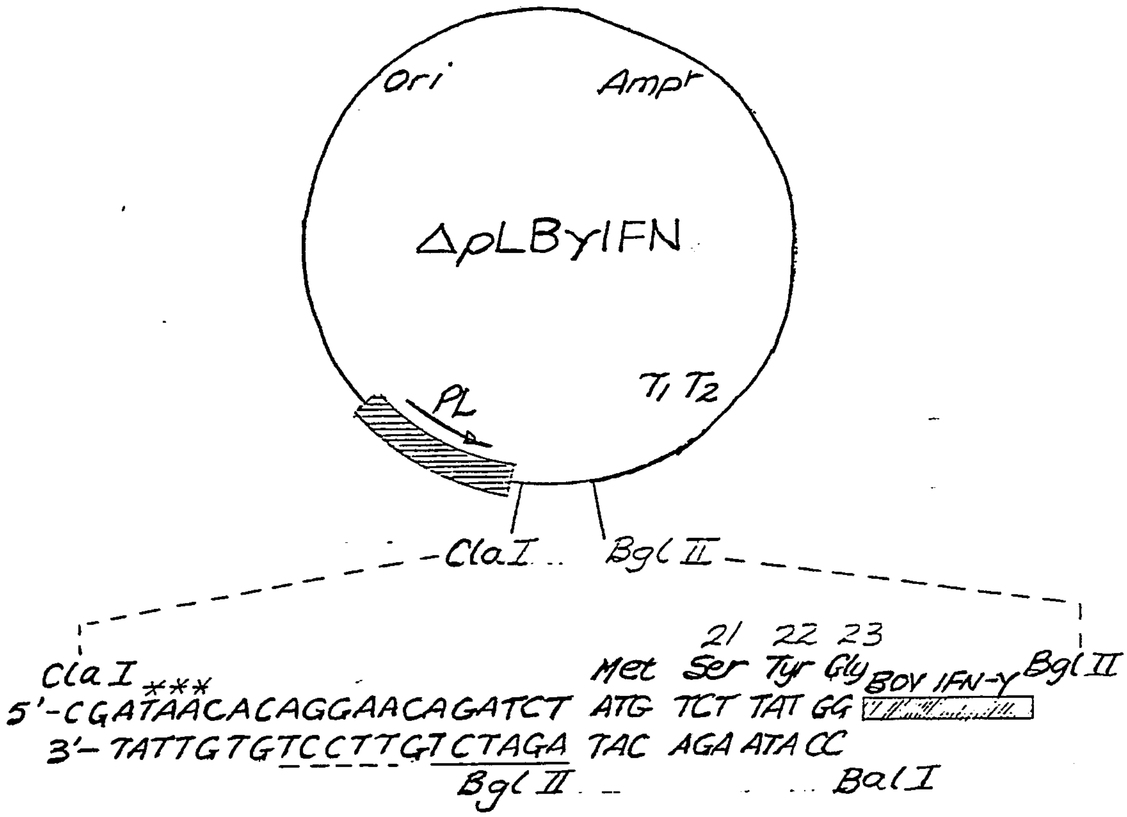


Fig. 3.

0230119

Kilburn & Strode

European Patent Attorneys
Chartered Patent Agents
Trade Mark Agents

Patents · Trade Marks
Designs · Copyright

**30 John Street**
**London WC1N 2DD**
**Telephone: (01) 242 8291**
**Telex: 23749**
**Fax: Gp II/III (01) 242 6630**
**Telegrams and Cables:**
**Bedkil, London WC1**

Our Ref: AGS/CB/P13084EP
Your Ref:
Date: 27th March 1987

European Patent Office
Patentlaan 2
Post Box 5818
2280 HV Rijswijk (ZH)
The Hague

Dear Sirs,

European Patent Application No. 86309440.5
Immunex Corporation

An obvious typographical error has been discovered in the numbering of the nucleotide sequence in Figure 1: nucleotides 410 et seq. were erroneously numbered as 420 et seq. This has been corrected on the enclosed clearer print of Figure 1, which is filed in triplicate. Clearer prints of Figures 2 and 3 are also enclosed, if they are necessary for printing.

A consequential amendment to page 17, line 32, of the description is necessary as a result of this correction to the drawings. The number of base pairs of the human IFN-gamma cDNA clone should, as a result of the corrected numbering be 692 instead of 702. Three copies of replacement page 17 showing this correction are enclosed.

If the receiving section is unwilling to let these corrections be made now, then we request that the receiving section pass on the details of the application to correct the errors to the examining division at the appropriate time.

Yours faithfully,

AG Sheard

A. G. Sheard
Authorised Representative

Enc.

Partners:
G.F. Arthur
M.J. Roos
A.J.M. Robinson
K.D.N. Kearney
R. Ashmead
N.R. Jennings
D.C. Rees
A.G. Sheard

Accounts:
R.W. Miller
Records:
W.D.D. Green
Manager:
K. Evans

0230119

European Patent Office

# EUROPEAN SEARCH REPORT

Application number

EP 86309440.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A2 - 0 088 622 (GENENTECH)<br>* Page 10, line 15 - page 12, line 30; page 35, line 12 - page 37, line 15; fig. 13 *<br>-- | 1 | C 12 N 15/00<br>C 07 H 21/04<br>C 12 N 1/20<br>C 07 K 15/26<br>C 12 P 21/00 |
| P,X | CHEMICAL ABSTRACTS, vol. 105, no. 9, September 1, 1986, Columbus, Ohio, USA<br>D.P.CERRETTI et al. "Cloning, sequence and expression of bovine interferon-gamma"<br>page 167, column 2, abstract-no. 73 588u<br>& J. Immunol. 1986; 136(12), 4561-4<br>* Totality *<br>-- | 1 | |
| P,X | CHEMICAL ABSTRACTS, vol. 104, no. 17, April 28, 1986, Columbus, Ohio, USA<br>M.J.STEINBECK et al. "Activation of bovine neutrophils by recombinant interferon-gamma"<br>page 526, column 2, abstract-no. 146 897k<br>& Cell Immunol. 1986; 98(1), 137-44<br>* Totality *<br>---- | 1 | TECHNICAL FIELDS SEARCHED (Int. Cl 4)<br>C 12 N<br>C 07 H<br>C 07 K<br>C 12 P |

The present search report has been drawn up for all claims

| Place of search | Date of completion of the search | Examiner |
|---|---|---|
| VIENNA | 03-03-1987 | BECKER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82